(19) Europäisches Patentamt
European Patent Office
Office européen des brevets

(11) Publication number: **0 126 343 A1**

(12) **EUROPEAN PATENT APPLICATION**

(21) Application number: **84104878.8**

(22) Date of filing: **01.05.84**

(51) Int. Cl.³: **C 07 D 487/04**, C 07 D 498/04, C 07 D 513/04, A 61 K 31/55 // (C07D487/04, 241/00, 223/00), (C07D487/04, 243/00, 241/00), (C07D498/04, 267/00, 241/00), (C07D513/04, 281/00, 241/00)

(30) Priority: **18.05.83 GB 8313689**
**18.05.83 GB 8313690**

(43) Date of publication of application: **28.11.84**
**Bulletin 84/48**

(84) Designated Contracting States: **CH DE FR GB IT LI NL**

(71) Applicant: **BEECHAM GROUP PLC, Beecham House Great West Road, Brentford Middlesex TW8 9BD (GB)**

(72) Inventor: **Bywater, Robert James, 1 Shelvers Way, Tadworth Surrey (GB)**
Inventor: **Newsome, Peter Martin, The Limes 20a York Road, Cheam Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

(54) **Treatment of diarrhoea.**

(57) A compound of formula (I)

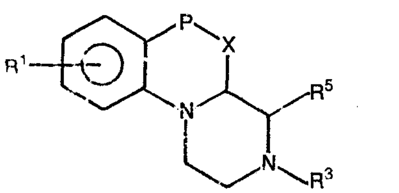

(I)

wherein (i) P is a bond, oxygen, sulphur, $NR^4$, methylene, ethylene or vinylene and X is

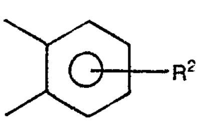

and $R^1$ and $R^2$ are each hydrogen, halogen, hydroxy, acyloxy, lower alkyl, lower alkoxy or trifluoromethyl
and $R^3$ is hydrogen, lower alkyl, lower aralkyl; aminoethyl or aminopropyl each optionally N-substituted by lower alkyl, or lower alkyl substituted nitrogen-containing heterocyclyl.
and $R^4$ is lower alkyl,
and $R^5$ is hydrogen,

or (ii) P is $-CR^6R^7$ and X is

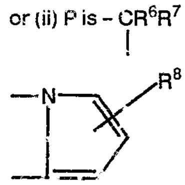

wherein the nitrogen is bonded to P.
and $R^1$ is hydrogen, lower alkyl, lower alkoxy, lower alkylthio, halogen or trifluoromethyl,
and $R^3$ is hydrogen, lower or higher alkyl, lower alkenyl or lower alkynyl, $C_{3-7}$ cycloalkyl, $C_{3-7}$ cycloalkenyl or lower alkyl substituted by $C_{3-7}$ cycloalkyl, hydroxy, amino, mono- or diloweralkylamino, carboxy, lower carbalkoxy, carbamoyl, mono- or di- lower alkyl-carbamoyl, phenyl, lower alkanoyl or phenylcarbonyl
and $R^5$, $R^6$ and $R^7$ are each hydrogen or lower alkyl
and $R^8$ is hydrogen, lower alkyl, carboxy, lower carbalkoxy, or lower alkyl substituted by hydroxy, amino, mono- or di-lower alkyl amino,
or a lower or higher alkanoyl, adamantoyl, carbamoyl, mono- or di- lower alkyl carbamoyl, $C_{3-7}$ cycloalkyl carbonyl or phenyl carbonyl derivative or a 2-N-oxide, 2-N-lower alkyl or phenyl lower alkyl quaternary derivative or a pharmaceutically acceptable salt thereof, is useful in treating diarrhoea or scours.

EP 0 126 343 A1

# TREATMENT OF DIARRHOEA

The present invention relates to the treatment of diarrhoea using a class of pentacyclic compounds and to pharmaceutical formulations for use in such treatment.

Compounds set out below are useful in treatment CNS disorders such as depression and anxiety. It has now suprisingly been found that these compounds are also useful in protecting young animals from death due to neonatal scours and in the treatment of scours and diarrhoea.

Accordingly the present invention provides a method for treating or preventing diarrhoea or scours which comprises administering to a human or non-human animal in need thereof an effective, non-toxic amount of a compound of formula (I):

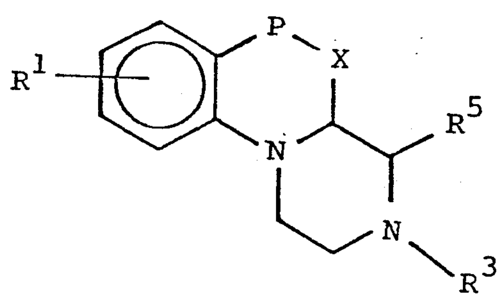

(I)

wherein (i) P is a bond, oxygen, sulphur, $NR^4$, methylene, ethylene or vinylene and X is

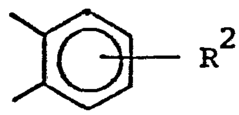

and $R^1$ and $R^2$ are each hydrogen, halogen, hydroxy, acyloxy, lower alkyl, lower alkoxy or trifluoromethyl

and $R^3$ is hydrogen, lower alkyl, lower aralkyl; aminoethyl or aminopropyl each optionally N-substituted by lower alkyl, or lower alkyl substituted nitrogen-containing heterocyclyl.

and $R^4$ is lower alkyl,

and $R^5$ is hydrogen,

or (ii) P is $-CR^6R^7$ and X is

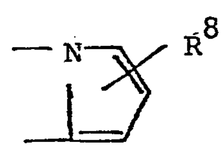

wherein the nitrogen is bonded to P.

and $R^1$ is hydrogen, lower alkyl, lower alkoxy, lower alkylthio, halogen or trifluoromethyl,

and $R^3$ is hydrogen, lower or higher alkyl, lower alkenyl or lower alkynyl, $C_{3-7}$ cycloalkyl, $C_{3-7}$ cycloalkenyl or lower alkyl substituted by $C_{3-7}$ cycloalkyl, hydroxy, amino, mono- or diloweralkylamino, carboxy, lower carbalkoxy,

carbamoyl, mono- or di- lower alkyl-carbamoyl, phenyl, lower alkanoyl or phenylcarbonyl
and $R^5$, $R^6$ and $R^7$ are each hydrogen or lower alkyl
and $R^8$ is hydrogen, lower alkyl, carboxy, lower carbalkoxy, or lower alkyl substituted by hydroxy, amino, mono- or di- lower alkyl amino,

or a lower or higher alkanoyl, adamantoyl, carbamoyl, mono- or di- lower alkyl carbamoyl, $C_{3-7}$ cycloalkyl carbonyl or phenyl carbonyl derivative or an 2-N-oxide, 2-N-lower alkyl or phenyl lower alkyl quaternary derivative or a pharmaceutically acceptable salt thereof.

Examples of salts are alkali metal and alkaline earth metal salts, especially sodium or disodium salts.

Acid addition salts may be formed from pharmaceutically or veterinarily acceptable inorganic and organic acids and include the salts of hydrochloric, hydrobromic, hydroiodic, nitric, sulphuric, citric, lactic, maleic, pamoic and tartaric acids.

As used herein the term 'lower' refers to radicals containing from 1 to 7 carbon atoms, preferably up to 4 especially one or two carbon atoms and 'higher' refers to radicals containing from 8 to 20, preferably 8 to 16 carbon atoms. The term 'nitrogen containing heterocyclyl' refers to, among others, pyridinyl, imidazolinyl, pyridazinyl, pyrimidinyl, pyrazinyl, morpholinyl, pyrrolyl and pyrrolidinyl.

Examples of compounds of formula (I) are

a) 1,2,3,4,10,14b-hexahydro-2-methyldibenzo[c,f]

pyrazinol[1,2-a]azepine,

b) 1,3,4,14b-tetrahydro-2-methyl-10H-pyrazino [1,2-a] -pyrrolo[2,1-c][1,4] benzodiazepine and

c) (+)-1,3,4,14b-tetrahydro-2-methyl-2H-dibenzo [b,f]-pyrazino[1,2-d][1,4]oxazepine and salts thereof.

Preferred salts of these compounds are the hydrochloride of compound (a), the maleate of compound (b) and the monohydrobromide of compound (c).

The compounds of formula (I) are known and they, and processes for their preparation, are disclosed and described in UK Patent No. 1 173 783, UK Patent No. 1 229 252 and US Patent No. 4 316 900.

It is believed that a suitable dose of a compound of formula (I) or a derivative or salt thereof, for use in the present treatment, would be in the range 0.001 to 100mg/kg, which dose may be repeated as and when required. Particularly suitable ranges include 0.01 to 10mg/kg and especially 0.01 to 5mg/kg. Nevertheless it will be appreciated that the dose used in practice will depend on the species to which the dose is administered and on the route of administration.

Pharmaceutical and veterinary compositions of a compound of formula (I) or a derivative or salt thereof (hereinafter referred to as the 'drug') will, of course, be adapted for administration to the humans or animals to be treated. Thus, for example, the composition may be a shaped composition, such as a bolus, tablet or capsule. In such cases the pharmaceutically or veterinarily acceptable carrier will be chosen from the usual range of lubricants, dispersants, binders, fillers and the like. When these

shaped compositions are for administration to cattle and pigs often they may for instance weigh at least 1 g, on occasions at least 2 g.

For administration to humans, especially children, the drug may suitably be presented as a syrup including suitable colouring and/or flavouring agents. Such syrups are conveniently presented in unit or multi-dose containers.

For veterinary use the composition may also be a dispersion or a solution of the drug in a suitable vehicle for use with an oral doser (this is a well known item of farm equipment, basically comprising a liquid reservoir, a mouthpiece adapted for insertion into animals mouths, and a pump mechanism whereby unit doses can be ejected from the reservoir through the mouthpiece). Conveniently the drug may be administered from an oral doser as an aqueous solution. Alternatively, the vehicle will be an oil or water based cream to ensure homogeneity of the unit doses administered.

The invention, therefore, also provides an oral doser containing a multi-dose of the drug in a veterinarily acceptable vehicle.

The drugs of the invention may also be added to the animal feed or drinking water. Thus the invention also provides animal feed or animal drinking water containing a compound of formula (I) or a derivative or salt thereof. It will be convenient to formulate these animal feed and drinking water compositions with a multi-dose of the drug so that the animal takes in an appropriate quantity of the drug along with its diet. It will also be convenient to present the composition of the invention as a premix for addition to the feed or drinking water.

With human babies or young animals, a particularly useful technique is to blend the drug with the milk provided for them.

The compositions of the invention may also be formulated for injection. In such cases the drug chosen is suitably dissolved in water for injection. Alternatively the drug may be administered in a solution used for parenteral fluid replacement therapy.

Treatment of diarrhoea and scours using the drug may be supplemented by oral rehydration therapy such as those described in U.K. Patent No. 1,581,826 and German Offenlegungsschrift No. 28 54 281, UK Patent Application No. 2 012 163A, US Patent No. 3 898 328, Nalin, D.R. and Cash, R.A., Bull. World Health Org., 43, 361 (1970), French Patent No. 2 467 599, UK Patent No. 1 465 308 and as described in 'Secretory Diarrhoea', Ed M. Field, J.S. Fordtran and S,G, Schultz, American Physiological Society, Maryland, 1980 pp 179-185 and Lancet, (1975) pp 79 and 80. Conveniently the drug may be administered with the oral rehydration formulation.

Accordingly the present invention provides, in a particular aspect, a formulation for treating diarrhoea which comprises an effective non-toxic amount of a compound of formula (I) or a derivative or salt thereof, as hereinbefore defined, and an oral rehydration composition comprising a pharmacologically acceptable aqueous solution containing at least 0.5% w/v of an actively absorbed monosaccharide, at least 25 mM sodium ions and having an osmolarity less than 500 m Osmolar.

Preferably the oral rehydration composition further comprises actively-absorbed amino acids and electrolytes.

The drug may be presented as a formulation containing one or more components of the oral rehydration composition for admixture with the remaining components.

Alternatively the drug may be provided separately and administered simultaneously or sequentially with the oral rehydration formulation.

Often it will be appropriate to include in the compositions a further medicine such as an antibacterial agent for example an antibiotic such as amoxycillin or neomycin or a sulphonamide such as sulfadoxin, an agent to alter intestinal motility such as loperamide or a material such as pectin.

The amount of drug administered must, of course, be sufficient to bring about the desired effect and will also depend on the body weight of the recipient and the chosen route of administration. Useful dosage units based on such dosage would contain from 0.01 mg to 500 mg of the drug, more suitably 0.01 mg to 250mg. Of course, it will be appreciated that many preferred compositions of the invention are in multi-dose form as, for the therapy of animals, it is often most desirable to be able rapidly to treat a number of animals. Such multi-dose compositions will contain, by way of example, at least 0.1 mg of the drug. Depending on the exact nature of the said

multi-dose composition, often it will contain at least 1 g of the drug, and on occasions as much as 5 g. Doses may be administered once or several times daily.

The following Formulations illustrate the invention but are not intended to limit the invention in any way.

As used in the Formulations the term 'Drug' refers to mianserin or a salt thereof, in particular the hydrochloride salt.

## Formulation of the Compounds for Veterinary Administration

### Formulation 1: Bolus

Boluses of the following composition may be prepared:-

| | |
|---|---|
| Drug | 50 mg |
| Microcrystalline cellulose | 500 mg |
| Corn starch | 250 mg |
| Magnesium stearate | 25 mg |
| Lactose, anhydrous | to 2500 mg |

The ingredients are passed through a 30 mesh stainless steel screen and blended in a suitable blender. The resultant compression mix is compressed directly on a tabletting machine to give tablets each containing 5 mg of the drug.

Formulation 2:

Oral Doser

1 Kg of the following composition may be prepared:-

| | % by wt. |
|---|---|
| Drug | 0.01 |
| Aluminium stearate | 6.0 |
| Sunflower oil | to 100 |

The aluminium stearate is dispersed with stirring in a portion of the sunflower oil heated to 115°C. The dispersion is added to the rest of the sunflower oil heated to 140°C. The gel is stirred at 130°C for 15 minutes and then allowed to cool without stirring to room temperature. The milled drug is dispersed in the cooled gel base and then passed through a colloid mill to produce a fine, homogenous dispersion. The dispersion is filled into plastic bottles fitted with a dosing pump.

Formulation 3

Injection

1 litre of the following composition may be prepared:-

| | % w/v |
|---|---|
| Drug | 0.05 |
| Sodium chloride | 0.5 |
| Water for injections | to 100 |

The drug and sodium chloride are dissolved in the water for injections and the solution is sterilised by membrane filtration and filled into glass ampoules.

Formulation 4

Soluble Powder

1 Kg of the following composition may be prepared:-

| | % by wt. |
|---|---|
| Drug | 0.2 |
| Lactose | to 100 |

The drug and lactose are sieved and mixed together in a suitable blender to give a homogenous powder. The powder is filled into jars. The powder is used at the rate 1 g per gallon of drinking water to medicate pigs.

Formulation 5

Oral Rehydration Formulation

1 kg of the following composition may be prepared by mixing together the ingredients in dry powder form:

| | |
|---|---|
| Glycine | 10.3 % |
| Dextrose (anhydrous) | 67.6 |
| Sodium Chloride | 14.3 |
| Potassium Dihydrogen Phosphate | 6.8 |
| Citric Acid | 0.8 |
| Tri-potassium Citrate | 0.2 |
| Drug | 0.003 |

60 g of the composition is then dissolved in 2 litres of water and fed to diarrhoeic calves.

Formulation 6

The following formulation may be prepared by the method set out below:-

| | |
|---|---|
| Drug | 0.02 % w/v |
| Bentone 38 (1) | 1.5 % w/v (ie 1.5 g/100ml) |
| Propylene Carbonate | 0.6 % w/v |
| Pharmasorb (2) | 10 % w/v |
| Phosphoric Acid (3) | 0.1 % w/v |
| Ampicillin Trihydrate | 6.0 % w/v as free acid |
| Soya-Bean Oil | to 100 % |

(1) Bentone 38 is an amide derivative of bentonite

(2) Pharmasorb is a brand of activated Attapulgite,

(3) The phosphoric acid is present to balance the alkaline pH of the Bentone.

The Bentone is dispersed in the soya-bean oil, and when thoroughly distributed, the propylene carbonate is added with high speed mixing, followed by colloid milling to produce the base. Into this base is first mixed the phosphoric acid, and then the pharmasorb, the penicillin, and the drug and the resultant suspension is then passed through a colloid mill once more.

0126343

CLAIMS

1. A pharmaceutical or veterinary composition for use in treating or preventing diarrhoea or scours, which comprises a compound of formula (I)

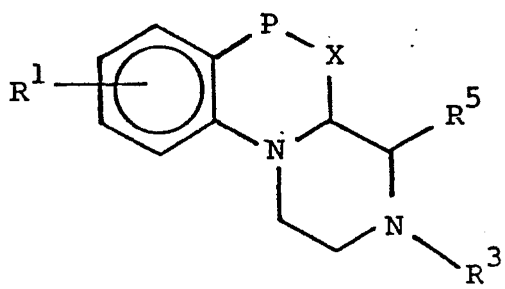

(I)

wherein (i) P is a bond, oxygen, sulphur, $NR^4$, methylene, ethylene or vinylene and X is

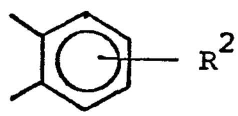

and $R^1$ and $R^2$ are each hydrogen, halogen, hydroxy, acyloxy, lower alkyl, lower alkoxy or trifluoromethyl

and $R^3$ is hydrogen, lower alkyl, lower aralkyl; aminoethyl or aminopropyl each optionally N-substituted by lower alkyl, or lower alkyl substituted nitrogen-containing heterocyclyl.

and $R^4$ is lower alkyl,

and $R^5$ is hydrogen,

or (ii) P is - $CR^6R^7$ and X is

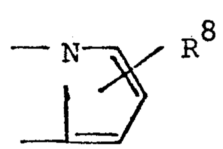

wherein the nitrogen is bonded to P.

and $R^1$ is hydrogen, lower alkyl, lower alkoxy, lower alkylthio, halogen or trifluoromethyl,
and $R^3$ is hydrogen, lower or higher alkyl, lower alkenyl or lower alkynyl, $C_{3-7}$ cycloalkyl, $C_{3-7}$ cycloalkenyl or lower alkyl substituted by $C_{3-7}$ cycloalkyl, hydroxy, amino, mono- or diloweralkylamino, carboxy, lower carbalkoxy, carbamoyl, mono- or di- lower alkyl-carbamoyl, phenyl, lower alkanoyl or phenylcarbonyl
and $R^5$, $R^6$ and $R^7$ are each hydrogen or lower alkyl

and $R^8$ is hydrogen, lower alkyl, carboxy, lower carbalkoxy, or lower alkyl substituted by hydroxy, amino, mono- or di- lower alkyl amino,

or a lower or higher alkanoyl, adamantoyl, carbamoyl, mono- or di- lower alkyl carbamoyl, $C_{3-7}$ cycloalkyl carbonyl or phenyl carbonyl derivative or an 2-N-oxide, 2-N-lower alkyl or phenyl lower alkyl quaternary derivatives or a pharmaceutically acceptable salts thereof, together with a pharmaceutically or veterinarily acceptable carrier.

2. A composition according to claim 1, in the form of a shaped composition for oral administration.

3. A composition according to claim 2 in the form of a bolus, tablet or capsule.

4. A veterinary composition according to claim 3 in which the bolus, tablet or capsule weighs at least 1g.

5. A composition according to claim 1 in the form of a premix for addition to the feed or drinking water of non-human animals.

6. A composition according to claim 1 in the form of an oral rehydration composition which additionally comprises a pharmacologically acceptable aqueous solution containing at least 0.5% w/v of an actively absorbed monosaccharide, and at least 25mM sodium ions and having an osmolarity less than 500m Osmolar.

7. A composition according to claim 6, further comprising actively - absorbed amino acids and electrolytes.

8. An oral doser containing a multi-dose of a compound of formula (I) as defined in claim 1 or a derivative or salt thereof, in a veterinarily acceptable vehicle.

9. A compound of formula (I) or a derivative or salt thereof for use in treating diarrhoea or scours.

10. The use of a compound of formula (I) or a derivative or salt thereof in treating diarrhoea or scours.

European Patent Office

# EUROPEAN SEARCH REPORT

0126343

Application number

EP 84 10 4878

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 173 783 (ORGANON LABORATORIES)<br>* Claim 1; page 1, lines 52-55 *<br>--- | 1 | C 07 D 487/04<br>C 07 D 498/04<br>C 07 D 513/04<br>A 61 K 31/55 //<br>(C 07 D 487/04<br>C 07 D 241/00<br>C 07 D 223/00 )<br>(C 07 D 487/04<br>C 07 D 243/00<br>C 07 D 241/00 )<br>(C 07 D 498/04<br>C 07 D 267/00<br>C 07 D 241/00 )<br>(C 07 D 513/04<br>C 07 D 281/00<br>C 07 D 241/00 ) |
| X | US-A-3 701 778 (W.J. VAN DER BURG)<br>* Claim 1; abstract *<br>----- | 1 | |
| | | | TECHNICAL FIELDS SEARCHED (Int. Cl. 3) |
| | | | A 61 K 31/55<br>C 07 D 487/04<br>C 07 D 498/04<br>C 07 D 513/04 |

The present search report has been drawn up for all claims

| Place of search | Date of completion of the search | Examiner |
|---|---|---|
| BERLIN | 07-08-1984 | KNAACK M |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document